# EUROPÄISCHE PATENTSCHRIFT

(11) **EP 2 519 621 B1**
(45) Veröffentlichungstag und Bekanntmachung des Hinweises auf die Patenterteilung: **02.12.2015**
(21) Anmeldenummer: 10813064.2
(22) Anmeldetag: 27.12.2010
(51) Int. Cl.: C11D 3/33, C11D 11/00, C11D 17/00, C07C 229/12, C07C 229/24

(54) **FESTSTOFF, DER GLUTAMINSÄURE-N,N-DIESSIGSÄURE ODER EIN SALZ DAVON ENTHÄLT SOWIE VERFAHREN ZUR HERSTELLUNG**
SOLID MATERIAL COMPRISING GLUTAMIC ACID OR SALT THEREOF AND PROCESS OF PREPARATION
MATIERE SOLIDE CONPRENANT DE L'ACIDE GLUTAMIQUE-N,N-DIACÉTIQUE OU UN SEL DE CELUI-CI ET PROCÉDÉ DE PRÉPARATION

(30) Priorität: 30.12.2009 DE 102009060814
(43) Veröffentlichungstag der Anmeldung: 07.11.2012
(73) Patentinhaber: BASF SE, 67056 Ludwigshafen (DE)
(72) Erfinder: BARANYAI, Andreas, 79423 Heitersheim (DE)
(86) Internationale Anmeldenummer: PCT/EP2010/007946
(87) Internationale Veröffentlichungsnummer: WO 2011/079940

(56) Entgegenhaltungen:
- EP-A2- 0 783 034
- WO-A1-2009/103822
- DE-A1- 19 649 681
- DE-A1- 19 937 345

## Beschreibung

Glutaminsäure-N,N-diessigsäure (GLDA) sowie Salze davon sind als Komplexbildner für Erdalkali- und Schwermetallionen in verschiedensten technischen Anwendungsgebieten von großem Interesse, wie z. B. in DE 196 49 681 A1 und in WO 2009/103822 beschrieben.

Komplexbildner für Erdalkali- und Schwermetallionen, wie sie beispielsweise in Wasch- und Reinigungsmitteln eingesetzt werden, werden üblicherweise in wässriger Lösung synthetisiert. Für bestimmte Anwendungsfälle werden sie in fester Form benötigt.

Übliche Verfahren zur Herstellung von Feststoffen aus Lösungen sind insbesondere Kristallisations- und Sprühtrocknungsverfahren. Bekannt ist, dass kristalliner Feststoff, wie er beispielsweise bei Verdampfungs- oder Kühlkristallisationsverfahren anfällt, Kristallwasser enthalten kann und unter Umgebungsbedingungen meist weniger hygroskopisch und lagerstabiler als amorpher Feststoff ist. Durch Sprühtrocknungsverfahren (z. B. im Sprühturm oder im Sprühwirbelbett) dagegen wird der Feststoff amorph erhalten. In dieser Form ist der Feststoff oft stark hygroskopisch und bei offener Lagerung unter Umgebungsbedingungen verliert er innerhalb kurzer Zeit die Rieselfähigkeit. Deshalb werden in der Literatur Maßnahmen beschrieben, um die Lagerstabilität von Sprühpulver zu erhöhen, z. B. die Kompaktierung oder Nachbehandlung von Buildern für Waschmittel mit Benzoesäure in der US 3, 932, 316.

Aufgabe der vorliegenden Erfindung war es, einen Feststoff bereitzustellen, der Glutaminsäure-N,N-diessigsäure (GLDA) und/oder Salze davon enthält und der z.B. für eine Verarbeitung und Anwendung vorzugsweise eine hinreichend geringe Hygroskopizität aufweist.

Die vorliegende Erfindung betrifft einen Feststoff, welcher z.B. für eine Verarbeitung und Anwendung vorzugsweise eine hinreichend geringe Hygroskopizität aufweist, der die folgenden Bestandteile umfasst:
a. einen Kern, der Glutaminsäure-N,N-diessigsäure (GLDA) und/oder ein Salz davon (insbesondere Glutaminsäure-N,N-diessigsäure (GLDA) und/oder ein Salz davon) enthält, und
b. eine Hülle, die eine Verbindung (oder mehrere verschiedene Verbindungen) der allgemeinen Formel I enthält, 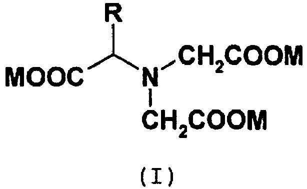 in der
   R für C₁- bis C₃₀-Alkyl oder C₂- bis C₃₀-Alkenyl, welche zusätzlich als Substituenten bis zu 5 Hydroxylgruppen, Formylgruppen, C₁- bis C₄-Alkoxygruppen, Phenoxygruppen oder C₁- bis C₄-Alkoxycarbonylgruppen tragen und durch bis zu 5 nicht benachbarte Sauerstoffatome unterbrochen sein können, für Alkoxylat-Gruppierungen der Formel

   -(CH₂)ₖ-O-(A¹O)ₘ-(A²O)ₙ-Y

   in der A¹ und A² unabhängig voneinander 1,2-Alkylengruppen mit 2 bis 4 C-Atomen bezeichnen, Y Wasserstoff, C₁- bis C₁₂-Alkyl, Phenyl oder C₁- bis C₄-Alkoxycarbonyl bedeutet und k für die Zahl 1, 2 oder 3 sowie m und n jeweils unabhängig voneinander für Zahlen von 0 bis 50 stehen, wobei die Summe aus m + n mindestens 4 betragen muss, für Phenylalkylgruppen mit 1 bis 20 C-Atomen im Alkyl, einen fünf- ode sechsgliedrigen ungesättigten oder gesättigten heterocyclischen Ring mit bis zu drei Heteroatomen aus der Gruppe Stickstoff, Sauerstoff und Schwefel, welcher zusätzlich benzanelliert sein kann, wobei alle bei den Bedeutungen für R genannten Phenylkerne und heterocyclischen Ringe noch zusätzlich als Substituenten bis zu drei C₁- bis C₄-Alkylgruppen, Hydroxylgruppen, Carboxylgruppen, Sulfogruppen und/oder C₁- bis C₄-Alkoxycarbonylgruppen tragen können, oder für einen Rest der Formel 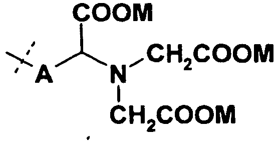 steht, wobei A eine C₁- bis C₁₂-Alkylen-Brücke oder eine chemische Bindung bezeichnet, und
   jedes M unabhängig voneinander Wasserstoff, Alkalimetall, Erdalkalimetall, Ammonium oder substituiertes Ammonium (z.B. organische Aminsalze) in den entsprechenden stöchiometrischen Mengen bedeutet (insbesondere bedeutet jedes M unabhängig voneinander Wasserstoff, Alkalimetall, Ammonium oder substituiertes Ammonium (z.B. organische Aminsalze) in den entsprechenden stöchiometrischen Mengen).

Die vorliegende Erfindung betrifft ferner ein Verfahren zur Herstellung des erfindungsgemäßen Feststoffes, welches dadurch gekennzeichnet ist, dass als Keim ein Feststoff vorgelegt wird, der Glutaminsäure-N,N-diessigsäure (GLDA) und/oder ein Salz davon (insbesondere Glutaminsäure-N,N-diessigsäure (GLDA) und/oder ein Salz davon) enthält (oder daraus besteht), und eine Sprühgranulation oder ein Coating (vorzugsweise in einer Wirbelschichtanlage) mit mindestens einer Verbindung der Formel I durchgeführt wird.

Wird das Kernmaterial (welches Glutaminsäure-N,N-diessigsäure (GLDA) und/oder ein Salz davon enthält) als Pulver eingesetzt, so wird dieses Pulver bei der Sprühgranulation vorzugsweise zunächst durch das Hüllenmaterial (welches eine Verbindung der allgemeinen Formel I enthält) zu größeren Teilchen verklebt (Granulation), bevor diese durch das Hüllenmaterial gecoated (umhüllt) werden. Wird das Kernmaterial in Form von größeren Granulaten vorgelegt, so werden diese Granulate bei der Sprühgranulation vorzugsweise direkt mit dem Hüllenmaterial gecoated (Coating).

Bevorzugt ist die Glutaminsäure-N,N-diessigsäure (GLDA) und/oder das Salz eine Verbindung der Formel (II)

M¹OOC-(CH₂)₂-CH(COOM¹)-N(CH₂COOM¹)₂ (II)

wobei M¹ Wasserstoff, Ammonium oder ein Alkalimetall (z.B. Natrium, Kalium) und/oder ein organisches Amin (organisches Aminsalz) ist. Insbesondere bevorzugt ist die L-Form davon. Weiterhin bevorzugt ist das Natriumsalz.

Insbesondere bevorzugt sind Verbindungen der Formel I, wie sie in DE 196 49 681 beschrieben sind.

Bei dem Verfahren der vorliegenden Erfindung werden Pulver und/oder Granulate des Kernmaterials in an sich üblicher Weise als Vorlage (Keime) in eine an sich bekannte geeignete Anlage (vorzugsweise eine Wirbelschicht-Sprühgranulationsanlage, z.B. vom Typ AGT 400 der Firma Glatt) gegeben und gewirbelt. Dabei sind Partikelgrößen von 30 µm - 300 µm (insbesondere 50 µm - 200 µm) für die Granulation und 200 µm - 1000 µm (insbesondere 300 µm - 1000 µm) für das Coating bevorzugt.

Vorzugsweise ist das Kernmaterial (GLDA enthaltende Material) trockenes Material, d.h. insbesondere weist das Kernmaterial einen Wassergehalt von weniger als 10% (insbesondere weniger als 5%) auf.

Vorzugsweise wird das Kernmaterial (GLDA enthaltende Material) in an sich üblicher Weise durch Kristallisation, Sprühtrocknung oder durch Wirbelschichtgranulation hergestellt.

Die Keime werden in einer Wirbelschicht im Schwebezustand (Fluidisierung) gehalten und bilden die Oberfläche für eine schichtweise Trocknung von verdüsten Tröpfchen, die mindestens eine Verbindung der Formel I enthalten. Die so erzeugten Partikel können bei der Herstellung über eine Granulation durch einen klassierenden Austrag flexibel - z.B. mit frei einstellbaren Partikelgrößen - ohne eine Unterbrechung des Trocknungsvorganges aus dem Trocknungsraum kontinuierlich entfernt werden. Bei der Herstellung durch reines Coating ist ein Batch Betrieb bevorzugt.

Zum Verfahren der Sprühgranulation siehe z.B.: H. Uhlemann, L. Mörl, "Wirbelschicht - Sprühgranulation" Springer - Verlag 2000 (ISBN 3-540-66985-X).

Das Verfahren der vorliegenden Erfindung ist dadurch gekennzeichnet, dass ein Feststoff, der Glutaminsäure-N,N-diessigsäure (GLDA) und/oder ein Salz davon enthält, als Keim vorgelegt wird und dann in an sich üblicher Weise eine Sprühgranulation/Coating mit mindestens einer Verbindung der Formel I, die vorzugsweise in Lösung (insbesondere in wässriger Lösung, bevorzugt ca. 35-43%ig, besonders bevorzugt ca. 35-42%ig, insbesondere bevorzugt ca. 40%ig) vorliegt, durchgeführt wird.

Vorzugsweise wird eine Sprühgranulation/Coating mit folgenden Parametern durchgeführt:
Bevorzugte Zulufttemperatur: 90-160°C,
bevorzugte Produkttemperatur: 50-120°C,
bevorzugte Ablufttemperatur: 40-110°C,
bevorzugte Temperatur der Speise: 40-100°C.

Bei dem erfindungsgemäßen Verfahren wird beispielsweise flüssiger Rohstoff (z.B. eine 35 bis 43%ige, bevorzugt eine 35-42%ige, insbesondere eine 40%ige wässrige Lösung einer Verbindung der Formel I) auf die im heißen Luftstrom wirbelnden Keime (enthaltend Glutaminsäure-N,N-diessigsäure (GLDA) und/oder ein Salz davon) aufgesprüht, der dadurch trocknet und die Keime beschichtet (gecoated) oder granuliert und anschließend gecoated.

Die Wirbelschichtanlage ist vorzugsweise ein Wirbelschicht Sprühgranulator, der beispielsweise mit einem Zyklon und / oder einem Filter und / oder einem Nasswäscher ausgestattet ist.

Als nichthygroskopisch bzw. hinreichend gering hygroskopisch wird hier insbesondere ein Feststoff bezeichnet, der bei der offenen Lagerung unter normalen Umgebungsbedingungen, z. B. 25°C und einer relativen Luftfeuchte von 76%, über einen Zeitraum von mindestens einem Tag, vorzugsweise einer Woche seine Konsistenz als (vorzugsweise rieselfähiges) Pulver oder Granulat bewahrt.

Der Kern des erfindungsgemäßen Feststoffs enthält Glutaminsäure-N,N-diessigsäure (GLDA) und/oder Salz davon, vorzugsweise eine Verbindung der allgemeinen Formel (II):

M¹OOC-(CH₂)₂-(CH(COOM¹)-N(CH₂COOM¹)₂ (II)

wobei M¹ Wasserstoff, Ammonium, ein organisches Amin oder ein Alkalimetall (vorzugsweise Natrium) ist. Besonders bevorzugt enthält der Kern L-Glutaminsäure-N,N-diessigsäure Tetranatriumsalz (C₉H₉NO₈Na₄, z.B. CAS No.: 51981-21-6). Vorzugsweise enthält das Kernmaterial Dissolvine® GL der Firma Akzo Nobel.

Gegebenenfalls kann der Kern einen oder mehrere weitere Komplexbildner für Erdalkali- und/oder Schwermetallionen wie z.B. Polymere, wie Polyacrylate oder sulfonierte Polymere (beispielsweise sulfoniertes Multipolymer wie Acusol 588 der Firma Room and Haas (Dow)) oder Ethylendiamintetraessigsäure (EDTA, beispielsweise Dissolvine NA der Firma Akzo Nobel), insbesondere als Tetranatriumsalz enthalten.

Besonders bevorzugt enthält der Kern ein GLDA Granulat oder Co-Granulat (d.h. ein Granulat mit Hilfsstoffen, die das Granulieren von GLDA vereinfachen, z.B. Polycarboxylate).

Im Kontext der vorliegenden Erfindung bezeichnet der Ausdruck GLDA vorzugsweise Glutaminsäure-N,N-diessigsäure oder eines der hierin beschriebenen Salze (z.B. das Tetranatriumsalz)

Der erfindungsgemäß hergestellte Feststoff umfasst als Hülle vorzugsweise im wesentlichen Verbindungen der Formel I, wobei geringe Mengen von Ausgangs- und/oder Nebenprodukten aus der Herstellung der Verbindungen der Formel I zusätzlich vorliegen können. Übliche Reinheiten für die Verbindungen I liegen, je nach angewandtem Syntheseverfahren, bei 70 bis 99,9 Gew.-%, insbesondere bei 80 bis 99,5 Gew.-%, jeweils bezogen auf den Feststoffgehalt.

Bei der vorliegenden Erfindung sind vorzugsweise solche Verbindungen der Formel I geeignet, bei denen R für C₁- bis C₂₀-Alkyl, C₂- bis C₂₀-Alkenyl oder einen Rest der Formel 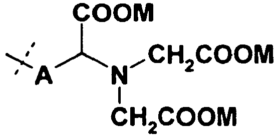 steht.

Besonders bevorzugt werden als Verbindung der Formel I α-Alanin-N,N-diessigsäure (R = CH₃, MGDA) und ihre Salze eingesetzt. Bevorzugt werden z.B. ihre Alkalimetall-, Ammonium- und substituierten Ammoniumsalze eingesetzt.

Als derartige Salze eignen sich vor allem die Natrium-, Kalium- und Ammoniumsalze, insbesondere das Trinatrium-, Trikalium- und Triammoniumsalz sowie organische Triaminsalze mit einem tertiären Stickstoffatom.

Als organischen Aminsalzen zugrunde liegende Basen kommen insbesondere tertiäre Amine, wie Trialkylamine mit 1 bis 4 C-Atomen in jedem Alkyl, wie Trimethyl- und Triethylamin, und Trialkanolamine mit 2 oder 3 C-Atomen im Alkanolrest, bevorzugt Triethanolamin, Tri-n-propanolamin oder Triisopropanolamin, in Betracht.

Als Erdalkalimetallsalze werden insbesondere die Calcium- und Magnesiumsalze eingesetzt.

Neben Methyl kommen für den Rest R als geradkettige oder verzweigte Alk(en)ylreste, vor allem C₂- bis C₁₇-Alkyl und -Alkenyl, hierbei insbesondere geradkettige, von gesättigten oder ungesättigten Fettsäuren abgeleitete Reste, in Betracht.

Beispiele für einzelne Reste R sind: Ethyl, n-Propyl, isoPropyl, n-Butyl, iso-Butyl, sec.-Butyl, tert.-Butyl, n-Pentyl, iso-Pentyl, tert.-Pentyl, Neopentyl, n-Hexyl, n-Heptyl, 3-Heptyl (abgeleitet von 2-Ethylhexansäure), n-Octyl, iso-Octyl (abgeleitet von iso-Nonansäure), n-Nonyl, n-Decyl, n-Undecyl, n-Dodecyl, iso-Dodecyl (abgeleitet von iso-Tridecansäure), n-Tridecyl, n-Tetradecyl, n-Pentadecyl, n-Hexadecyl, n-Heptadecyl, n-Octadecyl, n-Nonadecyl, n-Eicosyl und n-Heptadecenyl (abgeleitet von Ölsäure). Es können für R auch Gemische auftreten, insbesondere solche, die sich von natürlich vorkommenden Fettsäuren und von synthetisch erzeugten technischen Säuren, beispielsweise durch Oxosynthese, ableiten.

Als C₁- bis C₁₂-Alkylen-Brücken A dienen vor allem Polymethylengruppierungen der Formel (CH₂)ₖ, worin k eine Zahl von 2 bis 12, insbesondere von 2 bis 8 bezeichnet, d. h. 1,2-Ethylen, 1,3-Propylen, 1,4-Butylen, Pentamethylen, Hexamethylen, Heptamethylen, Octamethylen, Nonamethylen, Decamethylen, Undecamethylen und Dodecamethylen. Hexamethylen, Octamethylen, 1,2-Ethylen und 1,4-Butylen werden hierbei besonders bevorzugt. Daneben können aber auch verzweigte C₃-bis C₁₂-Alkylengruppen auftreten, z. B. -CH₂CH(CH₃)CH₂-, -CH₂C(CH₃)₂CH₂-, CH₂CH(C₂H₅) - oder CH₂CH(CH₃)-.

Die C₁- bis C₃₀-Alkyl- und C₂- bis C₃₀-Alkenylgruppen können bis zu 5, insbesondere bis zu 3 zusätzliche Substituenten der genannten Art tragen und durch bis zu 5, insbesondere bis zu 3 nicht benachbarte Sauerstoffatome unterbrochen sein. Beispiele für solche substituierten Alk (en) ylgruppen sind -CH₂OH, -CH₂CH₂OH, -CH₂CH₂-O-CH₃, -CH₂CH₂-O-CH₂CH₂-O-CH₃, -CH₂-O-CH₂CH₃, -CH₂-O-CH₂CH₂-OH, -CH₂-CHO, -CH₂-OPh, -CH₂-COOCH₃ oder -CH₂CH₂-COOCH₃.

Als Alkoxy(lat)-Gruppierungen kommen insbesondere solche in Betracht, bei denen m und n jeweils unabhängig voneinander für Zahlen von 0 bis 30, vor allem von 0 bis 15 stehen. A¹O und A²O bedeuten vorzugsweise von Butylenoxid und vor allem von Propylenoxid und von Ethylenoxid abgeleitete Gruppen. Von besonderem Interesse sind reine Ethoxylate und reine Propoxylate, aber auch Ethylenoxid-Propylenoxid-Blockstrukturen können auftreten.

Als fünf- oder sechsgliedrige ungesättigte oder gesättigte heterocyclische Ringe mit bis zu drei Heteroatomen aus der Gruppe Stickstoff, Sauerstoff und Schwefel, welche zusätzlich benzanelliert und durch die bezeichneten Reste substituiert sein können, kommen z.B. in Betracht:
Tetrahydrofuran, Furan, Tetrahydrothiophen, Thiophen, 2,5-Dimethylthiophen, Pyrrolidin, Pyrrolin, Pyrrol, Isoxazol, Oxazol, Thiazol, Pyrazol, Imidazolin, Imidazol, 1,2,3-Triazolidin, 1,2,3- und 1,2,4-Triazol, 1,2,3-, 1,2,4- und 1,2,5-Oxadiazol, Tetrahydropyran, Dihydropyran, 2H- und 4H-Pyran, Piperidin, 1,3- und 1,4-Dioxan, Morpholin, Pyrazan, Pyridin, α-, β- und γ-Picolin, α- und γ-Piperidon, Pyrimidin, Pyridazin, Pyrazin, 1,2,5-Oxathiazin, 1,3,5-, 1,2,3- und 1,2,4-Triazin, Benzofuran, Thionaphthen, Indolin, Indol, Isoindolin, Benzoxazol, Indazol, Benzimidazol, Chroman, Isochroman, 2H- und 4H-Chromen, Chinolin, Isochinolin, 1,2,3,4-Tetrahydroisochinolin, Cinnolin, Chinazolin, Chinoxalin, Phthalazin und Benzo-1,2,3-triazin.

N-H-Gruppierungen in den genannten heterocyclischen Ringen sollten möglichst in derivatisierter Form, etwa als N-Alkyl-Gruppierung (z.B. mit 1 bis 12 C-Atomen), vorliegen.

Bei Substitution an den Phenylkernen oder den heterocyclischen Ringen treten vorzugsweise zwei (gleiche oder verschiedene) oder insbesondere ein einzelner Substituent auf.

Beispiele für gegebenenfalls substituierte Phenylalkylgruppen und heterocyclische Ringe tragende Alkylgruppen für R sind Benzyl, 2-Phenylethyl, 3-Phenylpropyl, 4-Phenylbutyl, o-, m-oder p-Hydroxylbenzyl, o-, m- oder p-Carboxylbenzyl, o-, m-oder p-Sulfobenzyl, o-, m- oder p-Methoxy oder -Ethoxycarbonylbenzyl, 2-Furylmethyl, N-Methylpiperidin-4-ylmethyl oder 2-, 3- oder 4-Pyridinylmethyl.

Zur Substitution an Phenylkernen und auch an heterocyclischen Ringen werden bevorzugt wasserlöslich machende Gruppen wie Hydroxylgruppen, Carboxylgruppen oder Sulfogruppen eingesetzt.

Als Beispiele für die genannten C₁- bis C₄-, C₁- bis C₁₂- und C₁- bis C₂₀-Alkylgruppen sind auch die entsprechenden oben aufgeführten Reste für R zu verstehen.

Der erfindungsgemäße Feststoff eignet sich in besonderem Maße als Komponente für feste Wasch- und Reinigungsmittelformulierungen. Daher sind auch feste Wasch- und Reinigungsmittelformulierungen, die den erfindungsgemäßen Feststoff als Komplexbildner für Erdalkali- und Schwermetallionen in den hierfür üblichen Mengen neben anderen üblichen Bestandteilen solcher Formulierungen enthalten, Gegenstand der vorliegenden Erfindung. Zusammensetzungen und übliche Bestandteile derartiger fester Wasch- und Reinigungsmittelformulierungen sind dem Fachmann bekannt und brauchen daher hier nicht näher erläutert zu werden.

Das nachfolgende Beispiel soll die Erfindung näher erläutern. Als Verbindung I wurde α-Alanin-N,N-diessigsäure (Methylglycin-N,N-diessigsäure, "MGDA") Trinatriumsalz eingesetzt.

### Beispiele

Die folgenden Prozesse wurden auf einer Pilot Wirbelschicht-Sprühgranulationsanlage Typ AGT 400 der Firma Glatt durchgeführt, die mit einem Zyklon, einem Filter und einem Nasswäscher ausgerüstet war.

### Beispiel 1

Ziel dieses Versuchs war es, Granulate aus GLDA mit Trilon M flüssig (BASF) zu coaten. Die Zielkorngröße lag bei 200-1000µm.

### Versuchsdurchführung und Ergebnisse

Trilon M flüssig wurde auf 90-95°C erwärmt und auf das vorgelegte GLDA Granulat versprüht. Man erhielt Granulate im gewünschten Kornspektrum. Die Überkornfraktion wurde über ein 1000µm Sieb abgesiebt.

Bei folgenden Prozessparametern konnte das Coating stabil gefahren werden:
Zulufttemperatur 140°C
Zuluftmenge: 1400 m³/h
Produkttemperatur 100-104°C
Ablufttemperatur: 98-101°C
Sprühdruck: 2.0 bar
Sprührate: ca. 350-450g/min
Vorlagebehälter: 90°C

Nach Einstellung der oben genannten Prozessparameter konnte ein stabiler Prozess erzielt werden.

Durch eine anschließende Schutzsiebung über 1000µm konnten 15.9kg Granulat separiert werden. Die Überkornfraktion belief sich auf 6.0kg.

### Beispiel 2

Ziel dieses Versuchs war es, Co-Granulate aus GLDA und einem Polymer (Homopolymer Sokalan PA 30 CL (BASF)) mit Trilon M Liquid zu coaten. Zielkorngröße der gecoateten Granulate: 200-1000µm.

10kg Trilon M flüssig wurden auf 90-95°C erwärmt und auf das vorgelegte Co-Granulat (15 kg) aufgesprüht. Man erhielt Granulate im gewünschten Kornspektrum. Die Überkornfraktion wurde über ein 1000µm Sieb abgesiebt.

Bei folgenden Prozessparametern konnte das Coating stabil gefahren werden:
Zulufttemperatur 130-140°C
Zuluftmenge: 1200 m³/h
Produkttemperatur 104-106°C
Ablufttemperatur: 100-102°C
Sprühdruck: 2.0 bar
Sprührate: ca. 100-150g/min
Vorlagebehälter: 95°C

Es wurden die folgenden Mustermengen hergestellt:
17.0kg Granulat (über 1000µm gesiebt) Die Überkornfraktion lag bei 5.1kg.

Nach Einstellung der oben genannten Prozessparameter konnte ein stabiler Prozess erzielt werden.

### Beispiel 3

Ziel dieses Versuchs war es, Co-Granulate aus GLDA und einem Polymer (Homopolymer Sokalan PA 30 CL (BASF)) mit Trilon M Liquid zu coaten. Zielkorngröße der gecoateten Granulate: 200-1000µm.

20kg Trilon M flüssig wurden auf 90-95°C erwärmt und auf das vorgelegte Co-Granulat (15 kg) aufgesprüht. Man erhielt Granulate im gewünschten Kornspektrum. Die Überkornfraktion wurde über ein 1000µm Sieb abgesiebt.

Bei folgenden Prozessparametern konnte das Coating stabil gefahren werden:
Zulufttemperatur 130-140°C
Zuluftmenge: 1200 m³/h
Produkttemperatur 104-106°C
Ablufttemperatur: 100-102°C
Sprühdruck: 2.0 bar
Sprührate: ca. 100-150g/min
Vorlagebehälter: 95°C

Es wurden die folgenden Mustermengen hergestellt:
20.3kg Granulat (über 1000µm gesiebt) Die Überkornfraktion lag bei 5.2kg.

Nach Einstellung der oben genannten Prozessparameter konnte ein stabiler Prozess erzielt werden.

## Patentansprüche

1. Feststoff, der die folgenden Bestandteile umfasst:
a. einen Kern, der Glutaminsäure-N,N-diessigsäure und/oder ein Salz davon enthält, und
b. eine Hülle, die eine Verbindung der allgemeinen Formel I enthält, 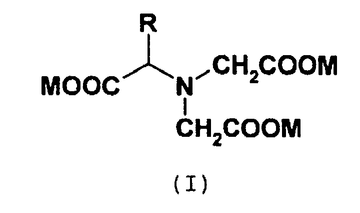 in der
R für C₁- bis C₃₀-Alkyl oder C₂- bis C₃₀-Alkenyl, welche zusätzlich als Substituenten bis zu 5 Hydroxylgruppen, Formylgruppen, C₁- bis C₄-Alkoxygruppen, Phenoxygruppen oder C₁- bis C₄-Alkoxycarbonylgruppen tragen und durch bis zu 5 nicht benachbarte Sauerstoffatome unterbrochen sein können, für Alkoxylat-Gruppierungen der Formel
-(CH₂)ₖ-O-(A¹O)ₘ-(A²O)ₙ-Y
in der A¹ und A² unabhängig voneinander 1,2-Alkylengruppen mit 2 bis 4 C-Atomen bezeichnen, Y Wasserstoff, C₁- bis C₁₂-Alkyl, Phenyl oder C₁- bis C₄-Alkoxycarbonyl bedeutet und k für die Zahl 1, 2 oder 3 sowie m und n jeweils unabhängig voneinander für Zahlen von 0 bis 50 stehen, wobei die Summe aus m + n mindestens 4 betragen muss, für Phenylalkylgruppen mit 1 bis 20 C-Atomen im Alkyl, einen fünf- oder sechsgliedrigen ungesättigten oder gesättigten heterocyclischen Ring mit bis zu drei Heteroatomen aus der Gruppe Stickstoff, Sauerstoff und Schwefel, welcher zusätzlich benzanelliert sein kann, wobei alle bei den Bedeutungen für R genannten Phenylkerne und heterocyclischen Ringe noch zusätzlich als Substituenten bis zu drei C₁- bis C₄-Alkylgruppen, Hydroxylgruppen, Carboxylgruppen, Sulfogruppen und/oder C₁- bis C₄-Alkoxycarbonylgruppen tragen können, oder für einen Rest der Formel 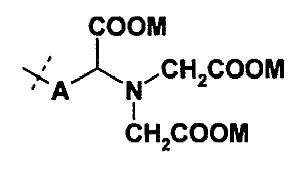 steht, wobei A eine C₁- bis C₁₂-Alkylen-Brücke oder eine chemische Bindung bezeichnet, und
jedes M unabhängig voneinander Wasserstoff, Alkalimetall, Erdalkalimetall, Ammonium oder substituiertes Ammonium (z.B. organische Aminsalze) in den entsprechenden stöchiometrischen Mengen bedeutet.

2. Feststoff nach Anspruch 1, wobei R für C₁- bis C₂₀-Alkyl, C₂- bis C₂₀-Alkenyl oder einen Rest der Formel 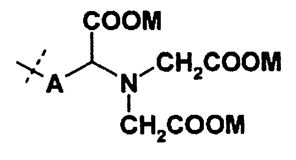 steht.

3. Feststoff nach Anspruch 1 oder 2, wobei die Hülle im wesentlichen aus α-Alanin-N,N-diessigsäure (MGDA) oder ihren Alkalimetall-, Ammonium- oder substituierten Aminsalzen besteht.

4. Feststoff nach einem der Ansprüche 1 bis 3, wobei die Glutaminsäure-N,N-diessigsäure (GLDA) und/oder das Salz eine Verbindung der Formel (II)
M¹OOC-(CH₂)₂-CHCOOM¹)-N(CH₂COOM¹)₂ (II)
ist, wobei M¹ Wasserstoff, Ammonium und/oder ein Alkalimetall oder ein organisches Amin ist.

5. Verfahren zur Herstellung eines Feststoffes nach einem der Ansprüche 1 bis 4, **dadurch gekennzeichnet, dass** als Keim ein Feststoff, der Glutaminsäure-N,N-diessigsäure (GLDA) und/oder ein Salz davon enthält, vorgelegt wird, und eine Sprühgranulation mit mindestens einer Verbindung der Formel I durchgeführt wird.

6. Verfahren nach Anspruch 5, **dadurch gekennzeichnet, dass** für die Sprühgranulation eine wässrige Lösung einer Verbindung der Formel I verwendet wird.

7. Feste Wasch- und Reinigungsmittelformulierungen, enthaltend einen Feststoff nach einem der Ansprüche 1 bis 4 als Komplexbildner für Erdalkali- und Schwermetallionen.

## Claims

1. A solid which comprises the following constituents:
a. a core which comprises glutamic acid-N,N-diacetic acid and/or a salt thereof, and
b. a coating which comprises a compound of the general formula I 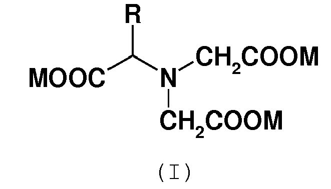 in which
R is C₁- to C₃₀-alkyl or C₂- to C₃₀-alkenyl, which additionally carries, as substituents, up to 5 hydroxyl groups, formyl groups, C₁- to C₄-alkoxy groups, phenoxy groups or C₁- to C₄-alkoxycarbonyl groups and can be interrupted by up to 5 nonadjacent oxygen atoms, is alkoxylate groups of the formula
-(CH₂)ₖ-O-(A¹O)ₘ-(A²O)ₙ-Y
in which A¹ and A², independently of one another, are 1,2-alkylene groups having 2 to 4 carbon atoms, Y is hydrogen, C₁- to C₁₂-alkyl, phenyl or C₁- to C₄-alkoxycarbonyl, and k is the number 1, 2 or 3, and m and n, in each case independently of one another, are numbers from 0 to 50, where the sum of m + n must be at least 4, is phenylalkyl groups having 1 to 20 carbon atoms in the alkyl, a five- or six-membered unsaturated or saturated heterocyclic ring having up to three heteroatoms from the group nitrogen, oxygen and sulfur, which can additionally be benzo-fused, where all of the phenyl rings and heterocyclic rings specified in the meanings for R can also additionally carry, as substituents, up to three C₁- to C₄-alkyl groups, hydroxyl groups, carboxyl groups, sulfo groups and/or C₁- to C₄-alkoxycarbonyl groups, or is a radical for the formula 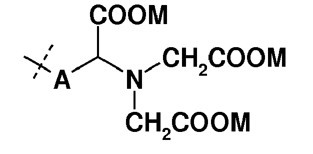 where A is a C₁- to C₁₂-alkylene bridge or a chemical bond, and
each M, independently of the others, is hydrogen, alkali metal, alkaline earth metal, ammonium or substituted ammonium (e.g. organic amine salts) in the corresponding stoichiometric amounts.

2. The solid according to claim 1, where R is C₁- to C₂₀-alkyl, C₂- to C₂₀-alkenyl or a radical of the formula

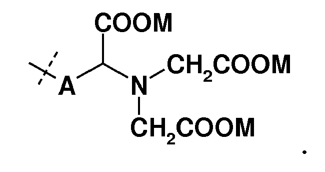

3. The solid according to claim 1 or 2, where the coating consists essentially of α-alanine-N,N-diacetic acid (MGDA) or its alkali metal, ammonium or substituted amine salts.

4. The solid according to any one of claims 1 to 3, where the glutamic acid-N,N-diacetic acid (GLDA) and/or the salt is a compound of the formula (II)
M¹OOC-(CH₂)₂-CH(COOM¹)-N(CH₂COOM¹)₂ (II)
where M¹ is hydrogen, ammonium and/or an alkali metal or an organic amine.

5. The method for producing a solid according to any one of claims 1 to 4, wherein the seed initially introduced is a solid which comprises glutamic acid-N,N-diacetic acid (GLDA) and/or a salt thereof, and a spray-granulation with at least one compound of the formula I is carried out.

6. The method according to claim 5, wherein an aqueous solution of a compound of the formula I is used for the spray-granulation.

7. A solid detergent or cleaner formulation comprising a solid according to any one of claims 1 to 4 as complexing agent for alkaline earth metal ions and heavy metal ions.

## Revendications

1. Matière solide, qui comprend les composant suivants :
a. un noyau qui contient de l'acide glutamyl-N,N-diacétique et/ou un sel de celui-ci, et
b. une enveloppe qui contient un composé de formule générale I, 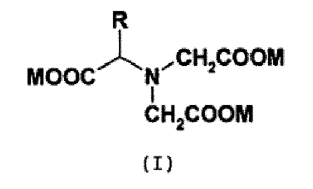 dans laquelle
R représente un groupe alkyle en C₁ à C₃₀ ou un groupe alcényle en C₂ à C₃₀, qui peuvent porter en plus comme substituants jusqu'à 5 groupes hydroxy, groupes formyle, groupes alcoxy en C₁ à C₄, groupes phénoxy ou groupes alcoxy(C₁ à C₄)carbonyle et être interrompus par jusqu'à 5 atomes d'oxygène non contigus, représente des groupements alcoxylate de formule
-(CH₂)ₖ-O-(A¹O)ₘ-(A²O)ₙ-Y
dans laquelle A¹ et A² représentent indépendamment l'un de l'autre des groupes 1,2-alkylène ayant de 2 à 4 atomes de carbone, Y représente un atome d'hydrogène, un groupe alkyle en C₁ à C₁₂, phényle ou alcoxy(C₁ à C₄)carbonyle et k représente le nombre 1, 2 ou 3 et m et n représentent chacun indépendamment l'un de l'autre des nombres de 0 à 50, la somme de m + n devant valoir au moins 4, représente des groupes phénylalkyle ayant de 1 à 20 atomes de carbone dans le fragment alkyle, un cycle hétérocyclique à cinq ou six chaînons, saturé ou insaturé, comportant jusqu'à trois hétéroatomes choisis dans le groupe constitué par les atomes d'azote, d'oxygène et de soufre, qui peut en outre être condensé avec un noyau benzénique, tous les cycles hétérocycliques et noyaux phényle nommés dans les significations de R pouvant encore porter en plus comme substituants jusqu'à trois groupes alkyle en C₁ à C₄, groupes hydroxy, groupes carboxy, groupes sulfo et/ou groupes alcoxy(C₁ à C₄)carbonyle, ou représente un radical de formule 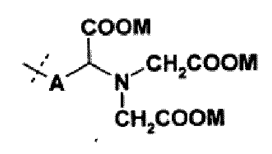
dans laquelle A représente un pont alkylène en C₁ à C₁₂ ou une liaison chimique, et
chaque M représente chaque fois indépendamment un atome d'hydrogène, un métal alcalin, un métal alcalino-terreux, un ion ammonium ou ammonium substitué (par exemple des sels d'amines organiques) en les quantités stoechiométriques correspondantes.

2. Matière solide selon la revendication 1, dans laquelle R représente un groupe alkyle en groupe alkyle en C₁ à C₂₀, alcényle en C₂ à C₂₀ ou un radical de formule

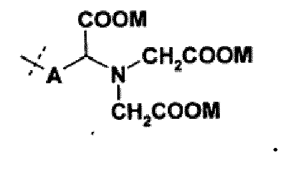

3. Matière solide selon la revendication 1 ou 2, dans laquelle l'enveloppe est essentiellement constituée d'acide α-alanine-N,N-diacétique (MGDA) ou de ses sels de métaux alcalins, d'ammonium ou d'amines substituées.

4. Matière solide selon l'une quelconque des revendications 1 à 3, dans laquelle l'acide glutamyl-N,N-diacétique (GLDA) et/ou le sel est un composé de formule (II)
M¹OOC-(CH₂)₂-CH(COOM¹)-N(CH₂COOM¹)₂ (II)
M¹ étant un atome d'hydrogène, un ion ammonium et/ou un métal alcalin ou une amine organique.

5. Procédé pour la préparation d'une matière solide selon l'une quelconque des revendications 1 à 4, **caractérisé en ce qu'**on dispose au préalable en tant que germe un solide qui contient de l'acide glutamyl-N,N-diacétique (GLDA) et/ou un sel de celui-ci, et on effectue une granulation par pulvérisation avec au moins un composé de formule I.

6. Procédé selon la revendication 5, **caractérisé en ce que** pour la granulation par pulvérisation on utilise une solution aqueuse d'un composé de formule I.

7. Compositions solides de produits de lavage et de nettoyage, contenant une matière solide selon l'une quelconque des revendications 1 à 4 en tant que complexant pour ions de métaux alcalino-terreux et de métaux lourds.
